# EUROPEAN PATENT APPLICATION

(11) **EP 4 644 538 A1**
(43) Date of publication of application: **05.11.2025**
(21) Application number: 25169607.6
(22) Date of filing: 10.04.2025
(51) Int. Cl.: C12N 5/0783, A61K 40/11, A61K 40/31, A61K 40/40, B01D 15/38, C12M 3/00, C12M 1/00, C12M 1/12, C12N 5/10

(54) **APPARATUS AND METHODS FOR T-CELL SEPARATION, ACTIVATION, TRANSDUCTION AND EXPANSION**

(30) Priority: 02.05.2024 US 202418653747
(71) Applicant: Southwest Research Institute, San Antonio, Texas 78238-5166 (US)
(72) Inventor: LING, Jian, Spring Branch, Texas, 78070 (US); FERNANDEZ, Angelica, San Antonio, Texas, 78238 (US)
(74) Representative: Lucke, Andreas

(57) **Abstract**

An apparatus and method for T-cell separation, activation, transduction and expansion. Three-dimensional (3D) bioreactors may be employed that include antibody coatings. Such 3D bioreactors can be employed for T-cell separation from peripheral blood mononuclear cells including attachment of T-cells to the 3D bioreactor surface for activation and transduction by lentivirus vectors to produce CAR T-cells. The CAR T-cells can then be expanded in a separate downstream bioreactor therein providing a scalable automated system.

## Description

This invention was made with government support under FDA Contract No. 75F40119C10158. The government has certain rights in the invention.

### FIELD

The present invention relates to an apparatus and method for T-cell separation, activation, transduction and expansion. Three-dimensional (3D) bioreactors may be employed that include antibody coatings. Such 3D bioreactors can be employed for T-cell separation from peripheral blood mononuclear cells including attachment of T-cells to the 3D bioreactor surface for activation and transduction by lentivirus vectors to produce CAR T-cells. The CAR T-cells can then be expanded in a separate downstream bioreactor therein providing a scalable automated system.

### BACKGROUND

Chimeric Antigen Receptor (CAR) T-cell therapy is considered a revolutionary approach that relies upon a patient's own immune cells to cure cancer. Five (5) CAR T-cell based therapies have gained FDA approval in the last four years. Numerous clinical trials are in progress. However, consistent, scalable and cost-effective manufacturing remains an on-going goal of CAR T-cell based therapies. Currently, the relatively high cost of CAR T-cell based therapies limits the number of patients who might benefit from this new cancer therapy and the biotechnology industry remains active in its pursuit of new platforms and protocols to reduce CAR T-cells.

U.S. Patent No. 10,988,724, entitled *Three-Dimensional Bioreactors For Cell Expansion And Related Applications,* describes a three-dimensional bioreactor that is composed interconnected voids with a plurality of pore openings between such voids.

U.S. Patent No. 11,149,244, entitled *Three-Dimensional Bioreactor For T-Cell Activation And Expansion For Immunotherapy,* describes a method for T-cell expansion that relies upon a 3D bioreactor comprising a plurality of voids having a surface area for cell expansion. A polydopamine coating is applied to the bioreactor surface, a tetrameric protein is then attached to the polydopamine coating, one or more biotinylated antibodies are immobilized on the tetrameric protein. One then flows T-cells through the 3D bioreactor having T-cell surface receptors that bind to the biotinylated antibodies and are activated. One then exposes the T-cell to a perfusion media containing a signaling molecule to promote T-cell expansion.

U.S. Patent No. 11,447,731, entitled *Three-Dimensional Bioreactors,* describes a three-dimensional bioreactor for growth of cells also composed of interconnected voids with a plurality of pore openings between the voids, where the bioreactor is coated with substituted or unsubstituted poly(p-xylylene).

U.S. Publication No. 20210317396 entitled *Three-Dimensional Bioreactor For Viral Vector Production* describes a three-dimensional bioreactor with a plurality of interconnected voids with a plurality of pore openings between the voids, including seeding the bioreactor with viral vector producing cells and flowing a perfusion medium through the bioreactor and promoting viral vector cell expansion.

U.S. Publication No. 20230139619 entitled *Devices For Cell Separation,* describes a device for cell separation that includes a plurality of non-random solid geometrical structures and optionally a plurality of non-random solid interconnecting elements between such structures. The surfaces of such device may be coated and functionalized to allow for selective ligand or cell binding and provide a method to separate one or more targeted cells from a plurality of cells.

A need nonetheless remains to provide both methods and devices to improve cellular expansion and in particular T-cell expansion via improved bioreactor designs. More specifically, newer perfusion-based and scalable bioreactor systems that facilitate an integrated T-cell separation, activation, transduction and expansion protocol.

### SUMMARY

A method for separating, activating, transducing and expansion of T-cells comprising:
**a.** supplying a 3D bioreactor comprising:
   **i.** a plurality of voids having a diameter **D** and a plurality of pore openings between said voids having a diameter **d,** including a void surface area for coating, wherein 90% or more of said voids have a selected void volume (V) that does not vary by more than +/- 10.0% and 90% or more of said pore openings between said voids have a value of d that does not vary by more than +/- 10.0%; or
   **ii.** a plurality of solid geometrical structures having outer surfaces for coating, wherein 90% or more of said solid geometrical structures have a volume (V) that does not vary by more than +/- 10.0%;
**b.** coating said 3D bioreactor with antibodies;
**c.** binding T-cells to said antibodies;
**d.** activating said T-cells;
**e.** transducing the antibody bound T-cells by a transduction reagent; and
**f.** removing said transduced T-cells from said 3D bioreactor and transferring to a T-cell expansion bioreactor wherein said transduced T-cells undergo expansion.

An apparatus for separating, activating, transducing and expansion of T-cells comprising:
**a.** a 3D bioreactor comprising:
   **i.** a plurality of voids having a diameter **D** and a plurality of pore openings between said voids having a diameter **d,** including a void surface area for coating, wherein 90% or more of said voids have a selected void volume (V) that does not vary by more than +/- 10.0% and 90% or more of said pore openings between said voids have a value of d that does not vary by more than +/- 10.0%; or
   **ii.** a plurality of solid geometrical structures having outer surfaces for coating, wherein 90% or more of said solid geometrical structures have a volume (V) that does not vary by more than +/- 10.0%; and
**b.** a T-cell expansion bioreactor connected to said 3D bioreactor.

### BRIEF DESCRIPTION OF THE DRAWINGS

**FIG. 1A** illustrates a portion of a 3D bioreactor herein having a plurality of non-random voids and non-random pore openings.
**FIG 1B** illustrates, in cross-section, a non-random void.
**FIG. 2A** illustrates a portion of a 3D bioreactor herein having non-random solid geometrical structures and optional solid non-random interconnecting elements.
**FIG. 2B** also illustrates a portion of a 3D bioreactor herein having non-random solid geometrical structures and optional solid non-random interconnecting elements.
**FIG. 2C** provides yet another illustration of a portion of a 3D bioreactor herein having non-random solid geometrical structures and optional solid non-random interconnecting elements
**FIG. 3** illustrates a portion of a 3D bioreactor herein showing the exemplary use of non-random spheres and non-random interconnecting elements.
**FIG. 4** illustrates the bioreactor of either the first or second configuration positioned in a housing between an inlet and outlet for fluid flow.
**FIG. 5** illustrates a portion of the 3D bioreactor surface herein within the non-random voids shown in **FIG. 1A** or on the surface of the non-random geometrical structures shown in **FIG. 2A** wherein the surface included the immobilized particle-facilitated antibody coating.
**FIG. 6A** illustrates the 3D bioreactor as shown in **FIG. 1A** or **FIG. 2A** positioned within a housing for inflow of a mixed population of T-cells, B-cells, monocytes and NK cells.
**FIG. 6B** illustrates the placement of the bound/activated T-cells in the 3D bioreactor **34.**
**FIG. 6C** illustrates the integrated bioreactor system where the activated T-cells undergo transduction to form CAR T-cells which may then be dissociated from the antibody and flushed form the antibody-coated 3D reactor into a separate expansion reactor.
**FIG. 7A** illustrates a negative 3D bioreactor combined with a gas permeable rapid expansion reactor.
**FIG. 7B** illustrates a preferred method of using the apparatus in **FIG. 7A** for T-cell purification, activation, transduction, and expansion.
**FIG. 8A** illustrates the proliferation of cells using the 3D bioreactor versus the use of Dynabeads^{™} over the indicated time period.
**FIG. 8B** illustrates the average cell size over the indicated time period.

### DETAILED DESCRIPTION

The present invention is directed at a bioreactor system that provides a scalable automated system. Namely, a first 3D bioreactor that may be a "positive" or "negative" 3D bioreactor coated with antibodies which are utilized for T-cell separation or purification, activation and transduction. The first 3D bioreactor is then connected to and provides a T-cell output to a second bioreactor that is a non-adherent T-cell expansion reactor, that provides for T-cell expansion, namely multiplication and proliferation.

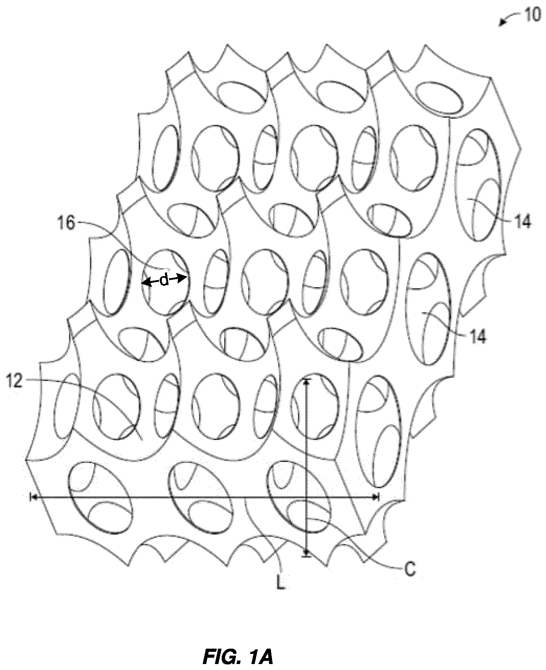

The first type of 3D reactor **10** for coating with antibodies is illustrated in **FIG. 1A****.** The 3D bioreactor shown in cut-away view includes a continuous interconnected void surface area **12** that includes a plurality of interconnected non-random voids **14** which are preferably of spherical shape with internal concave surfaces. By reference to non-random it be understood that one can now identify a targeted or selected number of voids in the 3D bioreactor that results in an actual repeating void size and/or geometry of a desired tolerance. The 3D bioreactor also includes non-random interconnected pore openings **16** as between the voids, which pore openings may also be of a desired tolerance. The 3D bioreactor also ultimately defines a layer of non-random voids (see arrow "L") and the multiple layers of the 3D bioreactor may then allow for identification of such non-random voids within a column (see arrow "C"). It is also useful to now note that such 3D bioreactor may be termed a "negative" bioreactor, since as noted, it relies about a plurality of interconnected non-random voids and non-random interconnected pore openings.

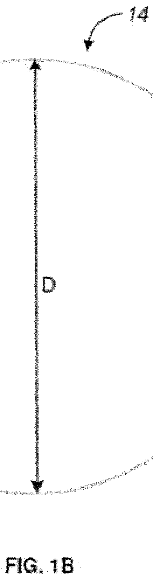

The 3D bioreactor in **FIG. 1A** is preferably such that the plurality of voids **14** have a diameter **D** (the longest distance between any two points on the internal void surface), the plurality of pore openings have a diameter **d** (the longest distance between any two points at the pore opening), where **D>d.** See **FIG. 1B** wherein the pore openings are not shown. In addition, 90.0 % or more of the voids, or even 95.0 % or more of such voids, or even 99.0% to 100% of such voids have a void volume (**V**) whose tolerance is such that it does not vary by more than +/- 10.0%, or +/- 5.0%, or +/- 2.5%, or +/- 1.0%, or +/- 0.5%, or +/- 0.1%.

Furthermore, 90.0% or more of the pore openings, or even 95.0% or more of the pore openings, or even 99.0% to 100% of the pore openings between the voids, indicate a value of **d** whose tolerance does not vary by more than +/- 10.0%, or +/- 5.0%, or +/- 2.5%, or +/- 1.0%, or +/- 0.5%, or +/- 0.1%. The diameter (**D**) of the voids preferably have a value in the range of 0.09 mm to 100.0 mm, including all individual values and increments therein. For example, the diameter of the voids may be in the range of 0.2 mm to 50.0 mm, or 0.2 mm to 50.0 mm, or 0.4 mm to 50.0 mm, or 0.4 mm to 25.0 mm. The diameter of the pore openings (**d**) are preferably in the range of 0.01 mm to 10.0 mm, including all values and increments therein. For example, the pore opening diameter (**d**) may be in the range of 0.05 mm to 2.0 mm or 0.1 mm to 2.0 mm.

The 3D bioreactor herein may also preferably be constructed in a second configuration comprising a plurality of non-random solid geometrical structures and optionally a plurality of interconnecting elements. This second configuration may therefore be understood as a "positive" 3D bioreactor. The solid geometrical structure may preferably include spheres, ovals, and/or polygonal shapes, thereby presenting an outer surface for production of CAR T-cells herein. As noted, such solid geometrical structures may optionally be connected via a plurality of solid interconnecting elements that may also assume a variety of geometrical shapes, including rod or columnar shape, oval shape, and/or polygonal type shape. Such solid interconnecting structure may also provide an outer surface for production of CAT T-cells. Both the solid geometrical structures and/or the solid interconnecting elements themselves are not necessarily completely solid and may contain partially hollow interiors to place nutrients and/or other reagents to improve the performance of such 3D bioreactor for production of CAT T-cells.

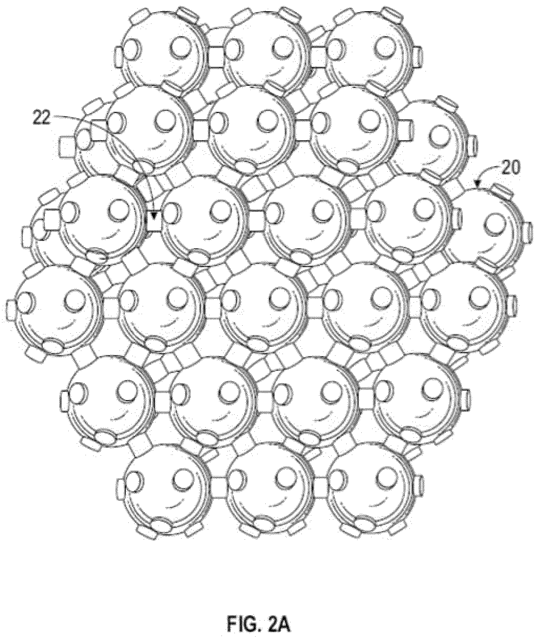

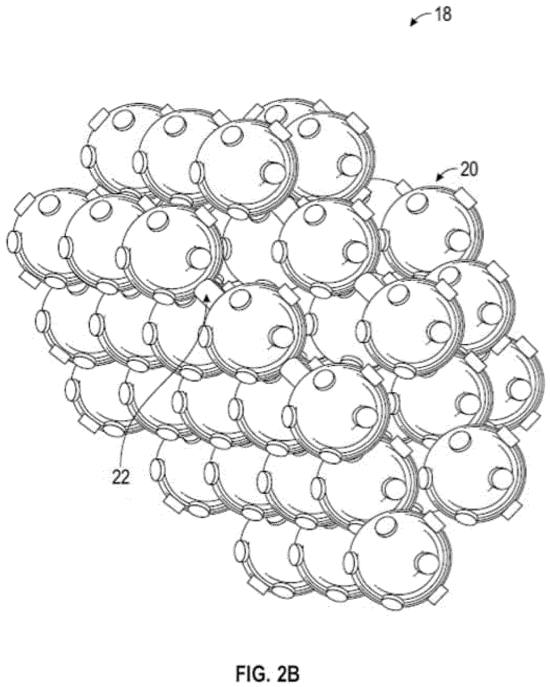

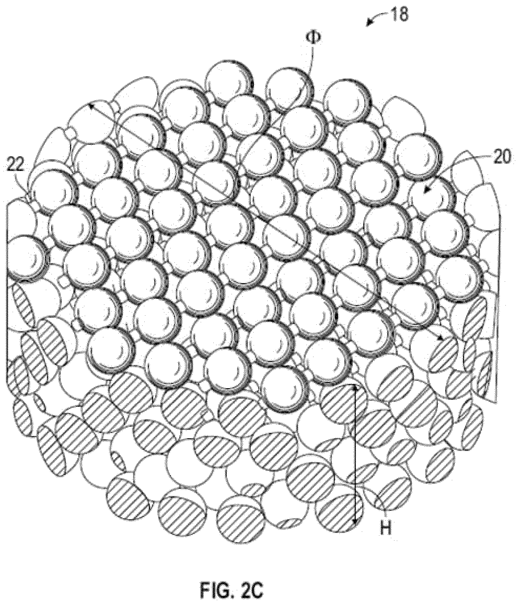

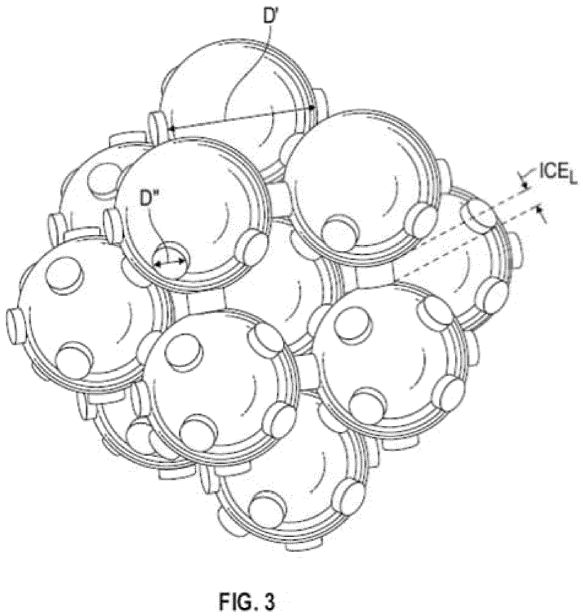

**FIGS. 2A** and **2B** illustrate the second configuration of a 3D bioreactor **18** herein where the solid non-random geometrical structures and optional solid non-random interconnecting elements preferably comprise spheres **20** and interconnecting rods **22.** As illustrated in **FIG. 2C****,** the 3D bioreactor **18** preferably has a diameter **Φ** in the range of 2.0 mm to 10,000 mm and a height **H** in the range of 1.0 mm to 5,000 mm. Preferably, the 3D bioreactor **18** has a **Φ/H** in the range of greater than 1:1. A portion of the 3D bioreactor **18** is illustrated in **FIG. 3** with respect to the exemplary use of spheres and the optional use of interconnecting rod elements. The non-random solid geometrical shapes herein preferably have a diameter **D'** (the longest distance between two points on the outer surface of the solid geometrical structure and through the structure interior) in the range of 2.0 µm to 25.0 mm, including all values and increments therein. Accordingly **D'** may have a value in the range of 200 µm to 25.0 mm, or 5.0 µm to 10.0 mm, or 5.0 µm to 6.0 mm, or 1.0 mm to 25.0 mm. The solid geometrical interconnecting elements (**ICE**) preferably have a diameter **D"** in the range of 1.0 µm to 12.5 mm, including all values and increments therein. Accordingly **D"** may preferably have a value of 1.0 µm to 3.0 mm. The length of the solid interconnecting elements (**ICE_{L}**) preferably has a value of 0.1 µm to 25.0 mm, including all values and increments therein. Accordingly, the **ICE_{L}** may have a value of 100.0 µm to 5.0 mm, or 100.0 µm to 3.0 mm. It is preferred that the diameter of the solid interconnecting structures (e.g., rods) are less than half of the value of the diameter of the solid geometrical shapes (e.g. spheres).

Similar to the first configuration of the 3D bioreactor, the second configuration can also be characterized by its overall non-random characteristics. That is, with respect to the solid geometrical structures (e.g., spheres **20**), 90% or more of such solid geometrical structures, or even 95.0% or more of such solid geometrical structures, or even 99.0% to 100% of such solid geometrical structures, define a volume whose tolerance is such that it does not vary by more than +/- 10.0%, or +/- 5.0%, or +/- 2.5% or +/- 1.0% or +/- 0.5% or +/0.1%. Similarly, with respect to the optional use of the solid interconnecting elements (e.g., rods 20), 90% or more of such solid interconnecting elements, or even 95.0% or more of such solid interconnecting elements, or even 99.0% to 100% of such solid interconnecting elements, define a volume whose tolerance is such that it does not vary by more than +/- 10.0%, or +/- 5.0%, or +/- 2.5% or +/- 1.0% or +/- 0.5% or +/-0.1%.

The 3D bioreactor device of the first configuration or second configuration is preferably made of biocompatible or bio-inert polymeric materials such as polystyrene, polycarbonate, acrylonitrile-butadiene-styrene (ABS), polylactic acid (PLA), polycaprolactone (PCL) used in FDM (fused deposition modeling) 3D printing technology. Reference to biocompatible or bio-inert should be understood as a material that is non-toxic to the culturing cells. In addition, the polymeric materials for the device of the first or second configuration are preferably selected from those polymers that at not susceptible to hydrolysis during cell cultivation, such that the amount of hydrolysis does not exceed 5.0 % by weight of the polymeric material present, more preferably it does not exceed 2.5 % by weight, and most preferably does not exceed 1.0 % by weight. The device of the first or second configuration may also be made of biocompatible photosensitive materials (e.g., Pro3Dure, Somos WaterShed XC 11122, etc.) used in SLA(stereolithography) and DLP (digital light processing) 3D printing technologies. Furthermore, the device of the first or second configuration may be formed of an interpenetrating polymer network (IPN). An IPN is reference to a polymer comprising two or more networks which are at least partially interlaced on a polymer scale but not covalently bonded to each other.

The 3D bioreactor devices of the first or second configuration herein are also preferably formed from material that indicates a Shore D Hardness of at least 10, or in the range of 10-95, and more preferably in the range of 45-95. In such regard, it is also worth noting that the devices herein preferably do not make use of a hydrogel type structure, which may be understood as a hydrophilic type polymeric structure, that includes some amount of crosslinking, and which absorbs significant amounts of water (e.g., 10-40 % by weight). It is also worth noting that the devices herein preferably do not make use of collagen, alginate, fibrin and other polymers that cells can easily be digested and undergo remodeling.

Furthermore, the 3D bioreactor devices herein of the first or second configuration are preferably made from materials that have a Tensile Modulus of at least 0.01 GPa. More preferably, the Tensile Modulus has a value that is in the range of 0.01 GPa to 20.0 GPa, at 0.01 GPa increments. Even more preferably, the Tensile Modulus for the material for devices herein are in the range of 0.01 GPa to 10.0 GPa or 1.0 GPa to 10 GPa. For example, with respect to the earlier referenced polymeric materials suitable for manufacture of the devices herein, polystyrene indicates a Tensile Modulus of about 3.0 GPa, polycarbonate at about 2.6 GPa, ABS at about 2.3 GPa, PLA at about 3.5 GPa and PCL at about 1.2 GPa.

The 3D bioreactor devices herein of either the first or second configuration with such preferred regular geometric characteristics and/or surface area are preferably fabricated by additive manufacturing technologies, such as fused deposition modeling FDM, selective laser sintering (SLS), stereolithography (SLA), digital light processing (DLP) 3D printing technologies, etc., according to computer generated designs made available by, e.g., a SolidWorks^{™} computer-aided design (CAD) program.

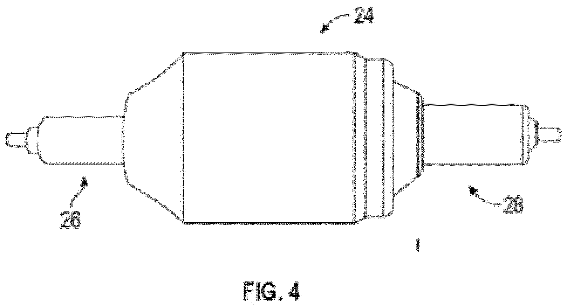

The 3D bioreactor devices of the first or second configuration may then be configured such that they amount to a fixed bed along with an inlet and outlet to allow for inflow and outflow of fluid. Reference is made to **FIG. 4** wherein the 3D bioreactor device of either the first or second configuration noted above may be positioned in a housing **24** and then placed between and inlet **26** and outlet **28** for which inflow and outflow of fluid may be provided containing cells for separation.

With regards to optional use of surface coating of the 3D bioreactor devices of either the first or second configuration, preferably, such coatings are those that may provide for affinity-based cellular capture. The coatings may therefore preferably comprise substituted or unsubstituted poly(p-xylylene) from the polymerization of parylene monomers, β-casein or polydopamine (PDA). Such coatings may preferably be present at a thickness in the range of 200 Angstroms to 100.0 µm.

Accordingly, the coating procedure preferably relies upon the use of parylene monomers, e.g., [2.2]paracyclophanes, that may be preferably functionalized with identified R₁, R₂, R₃ and R₄ groups according to the following general reaction scheme. It should be appreciated that in the scheme below, the start of polymerization is initiated by a ring opening at elevated temperature (~550 °C) in the low pressure gas phase remotely prior to deposition on the 3D device which is preferably maintained at relatively lower temperature (e.g., ≤ 100 °C):

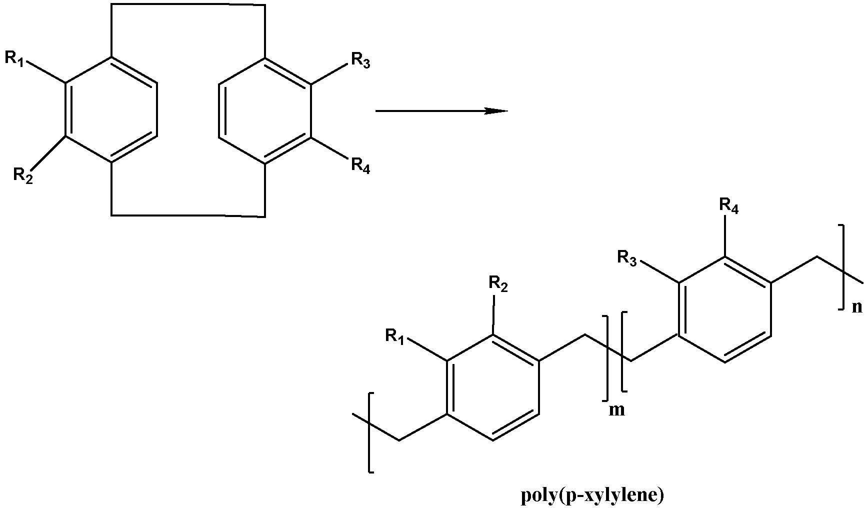

In the above, when one of the R groups per repeat unit "m" and/or repeat unit "n" is chlorine, and the other R groups are hydrogen, the above represents the polymerization of parylene C. It is a USP Class VI and ISO-10993-6 certified biocompatible material. The values of "m" and "n" of the identified crosslinked, repeating units are such that molecular weight values are relatively high, such as ~500,000. It is therefore contemplated that the use of the parylene monomers and ensuing polymeric coatings are such that one may now coat the devices of the above reference first or second configuration herein with an impermeable film. The film may preferably have a thickness between 200 Angstroms to 100.0 µm. It may be appreciated that R₁, R₂, R₃, and R₄ may be selected from hydrogen, a halogen (-Cl or -Br) as well as other functional groups such as amines (-NH₂), aliphatic aldehydes (-CHO), carboxylic acid functionality (-COOH), hydroxyl (-OH) or carboxylate functionality as in -C(O)CF₃. One may also initially coat with a first layer of impermeable parylene C followed by a coating of a different parylene, e.g., wherein R₁, R₂, R₃, and R₄ may then be selected from an amines (-NH₂) and/or aldehyde (-CHO) functionality. Accordingly, one may provide polymeric coatings for the devices herein of the first and/or second configuration, wherein the coating comprises a plurality of layers, each with its own particular and different chemical composition (i.e. the identity of at least one of R₁, R₂, R₃, and R₄ are different between at least two of the layers).

One preferred method of coating the surface of the devices herein with functionalized poly(p-xylylene) applies when one or more of the R₁, R₂, R₃ and/or R₄ groups noted above comprise ester carboxylic acid functionality. In such a case, one may utilize N-hydroxysuccinimide (NHS) to form an ester linkage. Next, NH₂-mPEG (methoxy terminated oligoethylene glycol) or NH₂-PEG-biotin may be covalently bonded to the device surface via the amine-NHS ester reaction to form an amide bond. Then, avidin or NeutrAvidin^{™} (deglycosylated native avidin protein) or streptavidin can be bound to the biotin. As avidin/ deglycosylated native avidin protein /streptavidin have four bonding sites, the remaining three sites are then available to bind biotinylated antibodies, such as anti-CD3 and anti-CD28 to capture T cells through surface receptors specific to these antibodies.

### Coating Of The 3D Bioreactors With Antibodies

The 3D bioreactors noted herein may be coated with antibodies. This may be achieved by directly coating the biocompatible or bio-inert polymeric materials noted above that are relied upon to form the 3D bioreactor and hydrophobic surface, with various antibodies, such as anti-CD2, anti-CD3, or anti-CD28 through hydrophobic-hydrophobic interactions. One may also first coat the 3D bioreactors with, e.g., a tetrameric protein and then with a biotinylated antibody that binds to the tetrameric protein. Alternatively, one may first coat the surfaces of the 3D bioreactors with a surface coating that comprises substituted or unsubstituted poly(p-xylylene) from the polymerization of parylene monomers, β-casein or polydopamine (PDA). Following such coating, the coatings themselves on the 3D bioreactor can be coated with one or more selected antibodies. For example, the polydopamine coating on the 3D bioreactor may itself be coated with a tetrameric protein following be coating with biotinylated antibodies. Or, one may initially coat the 3D bioreactors herein with poly(p-xylylene), followed by polydopamine, tetrameric protein and then biotinylated antibodies.

The 3D bioreactors herein may also more preferably be coated with antibody labelled particles on the 3D bioreactor surfaces as disclosed in U.S. Appl. No. 18/640,078. As noted, one may place antibody coatings directly on the internal surface of 3D bioreactors. The particles preferably include any biocompatible particle that can serve to immobilize an antibody. Accordingly, the particles may comprise silica (SiO₂) particles and/or biocompatible polymeric particles. Antibody labelled particles herein are therefore a reference to particles that have an antibody immobilized to the particle, which may preferably occur by covalent attachment or by, e.g., secondary interactions (e.g., hydrophobic-hydrophobic interactions). The particles preferably have a diameter of less than or equal to 1.0 µm. Accordingly, the particles preferably have a diameter in the range of 10 nm to 1000 nm (1.0 µm), including all individual values and increments therein.

The particles may therefore be preferably coated with a biotin binding molecule, such as a tetrameric protein (protein with quaternary structure), which includes avidin, streptavidin or deglycosylated native avidin protein. The particles with a biotin binding molecule may then be coated with biotinylated antibodies. More specifically, one can now immobilize biotinylated antibodies such as anti-CD3, anti-CD19, anti-CD22, anti-CD25 and anti-CD28 to the particle that has been coated with a biotin binding molecule via, e.g., the biotin-avidin/streptavidin binding mechanism. A biotinylated antibody is reference to the covalent attachment of any one or more of the identified antibodies to the biotin binding molecule now associated with the particle surface.

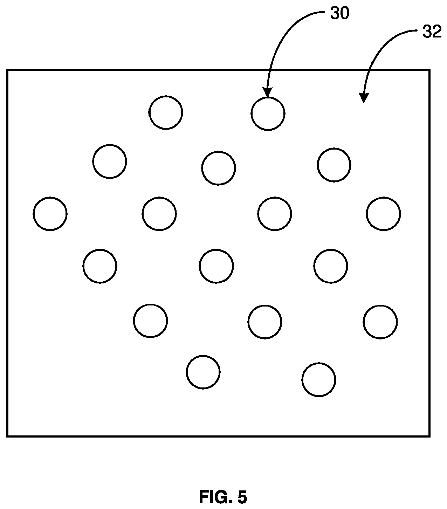

The antibody labelled particles now described herein provide relatively easy convection of fluid suspended particles into the 3D bioreactors herein with subsequent attachment to the surfaces of the 3D bioreactors. Such attachment to the 3D bioreactor surface is contemplated to occur through a hydrophobic-hydrophobic interaction. The immobilized antibody-labelled particles preferably provide an array of discrete antibody labelled particles on the 3D bioreactor surface. FIG. 5 illustrates a portion of the 3D bioreactor surface herein, wherein by controlling the number of antibody labelled particles **30** on the surface **32** of the 3D bioreactor, the average surface density of the antibody labelled particles can be determined for a relatively uniform coating. The 3D bioreactor surface in **FIG. 5** may be a portion of the 3D bioreactor surface within the non-random voids **14** shown in **FIG. 1A** or on the surface of the non-random geometrical structures **20** (e.g,. spheres) or non-random interconnecting elements (e.g. rods) shown in **FIG. 2A****.** The average surface density of the antibody labelled particles on the surface of the 3D bioreactor preferably falls in the range of 3.9x10⁵ particles/cm² to 3.2x10⁷ particles/cm². The average distance between the antibody labelled particles on the 3D bioreactor surface preferably falls in the range of 1.0 µm to 9.0 µm.

It may therefore be appreciated that the 3D bioreactors herein may be directly coated with antibodies. Or, the 3D bioreactors herein may first be coated with poly(p-xylylene), or β-casein or polydopamine (PDA) and then coated with antibodies. The 3D bioreactors herein may also be preferably coated with antibody labelled particles. Any one of these coated 3D bioreactors may now be advantageously integrated with a cell expansion device to provide a scalable automated system.

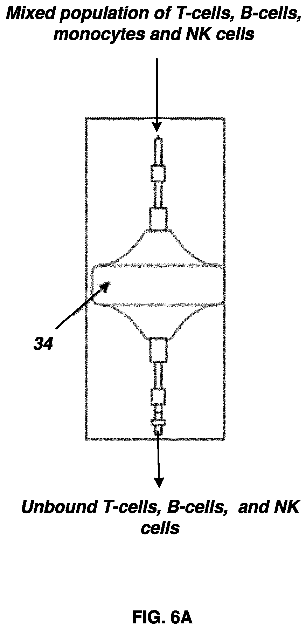

Attention is directed to **FIG. 6A** where an antibody coated 3D bioreactor **34** (as shown in **FIG. 1A** or **FIG. 2A**) is positioned within a housing for inflow and outflow of fluid so that it may receive a mixed population of T-cells, B-cells, monocytes and NK cells. More specifically, a mixed population of lymphocytes 70-90% (T-cells, B-cells, NK cells), 10-20% monocytes and 1-2% dendritic cells. The frequencies of cell types within the lymphocyte population include 70-85% CD3+ T-cells, 5-10% B-cells, and 5-20% NK cells. The CD3+ lymphocytes are composed of CD4+, CD8+ T-cells, in about a 2:1 ratio. The cell surface receptors on T-cells (e.g. CD3+, CD28+ coactivator receptor) can bind to the antibodies (e.g., anti-CD3, anti-CD28) immobilized on the 3D bioreactor surface.

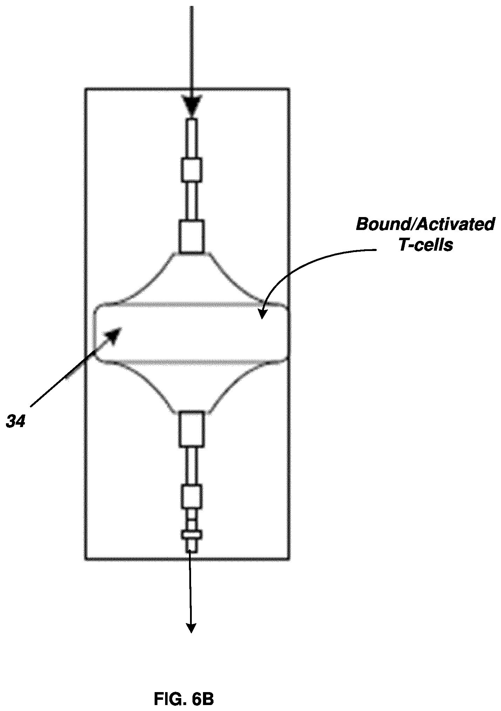

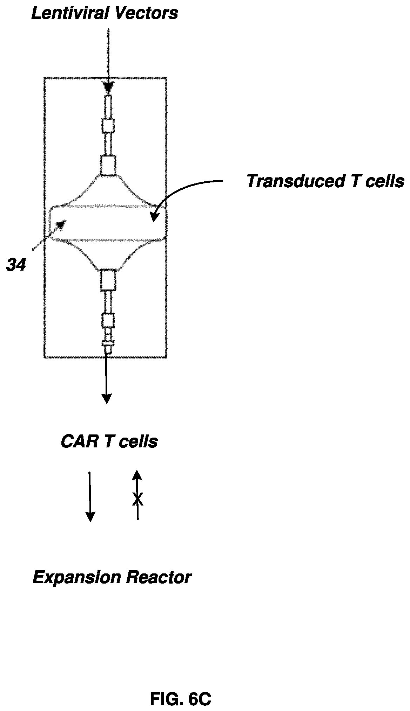

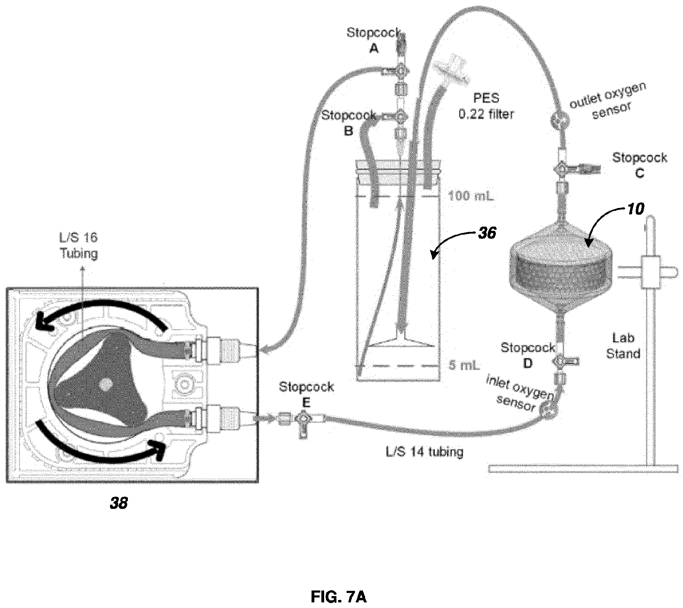

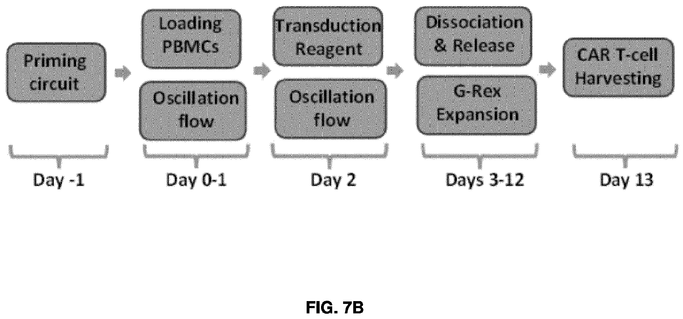

**FIG. 6B** shows the bound/activated T-cells on the 3D bioreactor **34.** As next shown in **FIG. 6C****,** an integrated bioreactor system is shown, where the activated T-cells may then undergo transduction which form CAR T-cells which may then be dissociated from the antibody and flushed out from the antibody-coated 3D bioreactor into a separate expansion reactor which provides oxygen and nutrition for CAR T-cell expansion. See also **FIG. 7****.** Preferred expansion reactors therefore include stirred tank bioreactors which rely upon mechanical agitation, wave-bioreactors that rely upon rocking motion, and a gas permeable rapid expansion bioreactor sold as the G-Rex^{™} cell culture bioreactor. The CAR T-cells flowing out of the 3D bioreactor are also prevented from recirculating back into the antibody-coated 3D bioreactor to avoid the restimulation of the CAR T-cells which can lead to apoptosis (cell death).

### Working Examples

### Working Example 1 - Coating Of 3D Bioreactor With Antibody Labelled Silica Particles

As disclosed in U.S. Appl. No. 18/640,078, silica particles having a 1.0 µm diameter were coated with streptavidin and then with a biotinylated anti-CD19, anti-CD22 and anti CD-25 antibodies. The antibody coated particles were suspended in 2.0 ml of a phosphate buffer solution (PBS) and circulated through a 3D bioreactor as illustrated and described in **FIGS. 2A-2C** and **3,** having a internal surface area of 100 cm². The circulation took place for about 16 hours at 2 °C to 8 °C. The flow rate was 0.1 mL/min. After coating, the unbound particles were flushed out by 10 ml of PBS flowing from the 3D bioreactor inlet to outlet at the rate of 1 mL/min. At the same flow rate a second 10 mL of PBS was flushed through the 3D bioreactor from outlet to inlet. The unbound particles flowing out of the reactor were collected and counted to determine the number of antibody coated particles remaining in the 3D bioreactor. **Table 1** below shows that an average of at least 98% of the antibody coated silica particles remained attached to the 3D bioreactor surface.

**Table 1**

| **Immobilization of Antibody Labelled 1.0 µm Silica Particles On 3D Bioreactor Surface** | | | |
|---|---|---|---|
| **Trial** | **Particles Used In Coating** | **Flushed Out Nonbound Particles** | **Percent Adhered To 3D Bioreactor Surface** |
| 1 | 9.20E+08 | 3.47E+06 | 99.62 |
| 2 | 9.20E+08 | 6.00+06 | 99.35 |
| 3 | 9.20E+08 | 3.18+07 | 97.38 |
| 4 | 9.20E+08 | 5.98E+06 | 99.49 |
| **Average** | 9.20E+08 | 1.18E+07 | 98.96 |
| **STD** | 1.00E+05 | 1.34E+07 | 1.06 |

### Working Example 2 - Coating Of 3D Bioreactor With Antibody Labelled Silica Particles

As disclosed in U.S. Appl. No. 18/640,078, a 3D bioreactor as illustrated and described in **FIG. 1A** having an internal surface area of 250 cm², and an internal fluid volume of about 13 mL was also coated with antibody silica coated particles. Namely, about 4.56x10⁹ streptavidin coated 1.0 µm silica particles (Bands Lab, Cat # CS1001) were added to a 1.5 ml Eppendorf tube. The particles were washed with sterilized PBS twice to remove any preservation agent such as sodium azide (NaN₃). After washing, the particles were first coated with 5µg of biotinylated sheep antimouse secondary antibody and the further coated with 15 µg/mL of mouse IgG anti-CD3 and 15 µg/mL of mouse IgG anti-CD28 antibodies. All coatings were carried out at room temperature for 1 hour.

The antibody-coated silica particles were suspended in 40 mL of PBS which was circulated through the 3D bioreactor at a flow rate of 0.1 mL/min. The perfusion-based particle coating was carried out for about 16 hours at 2 °C to 8 °C. After coating, 130 mL of PBS, or 10 times the 3D bioreactor internal void volume was flushed through the 3D bioreactor by gravity to eliminate nonbound particles. Typically, about 3x10⁶ particles were attached to the 3D bioreactor's 250 cm² internal surface generating an average distance between the antibody labelled particles on the 3D bioreactor surface, between two 1.0 µm diameter silica particles, of 1.88 µm.

### Working Example 3 - 3D Bioreactor In Combination With A Gas Permeable Rapid Expansion Bioreactor As A Scalable Automated System

As illustrated in **FIG. 7A****,** a negative 3D bioreactor **10** as disclosed herein, having a plurality of interconnected non-random voids and non-random interconnected pore openings, with an available surface area of 250 cm² and internal volume of about 13.0 mL, was combined with a gas permeable rapid expansion bioreactor 36 along with a peristaltic perfusion pump 38. The 3D bioreactor surface was coated with anti-CD3 and anti-CD28 antibody functionalized silica particles having a 1.0 µm diameter. It is worth noting that the 3D bioreactor 10 may also be a positive 3D bioreactor as described herein. The gas permeable rapid expansion bioreactor was a G-Rex^{™} 6M which can accommodate 5x10⁶ activated cells and potentially expand to 300 x 10⁶ to 400 x 10⁶ cells. During the initial loading of peripheral blood mononuclear cells (PBMCs), the medium volume in the G-Rex^{™} 6M is only about 5.0 mL. The PBMCs suspension is perfused thought the 3D bioreactor to facilitate interactions between the cells and the antibody coating to achieve T-cell binding and activation. During a 48 hour perfusion, the medium was aspirated from the bottom of the G-Rex 6M and circulated back into the 3D bioreactor from which it could be recirculated back into the G-Rex 6M.

After 48 hours the G-Rex 6M was first filled with fresh medium (~100 mL). The position of the medium intake was switched from the bottom of the gas permeable rapid expansion reactor 36 to the top of the reactor. Since cells flowing out from the 3D bioreactor 10 gravitationally settle to the bottom of the reactor, because of their higher density relative to the media, the media introduced from the top of the reactor will be cell free. In other words, the system prevents flushed cells from reentering the 3D bioreactor 10 to trigger restimulation which can result in cell apoptosis. Cells settling at the bottom of the reactor 36 received oxygen through an underlying gas permeable membrane. The supernatant media can then be refurbished as desired to induce continued proliferation with the oxygen and additional nutrition.

More preferably to the general procedures noted above, before loading the PBMCs, the system illustrated in **FIG. 7B** is first primed with 24 mL media to pre-oxygenate the media and to remove any air bubbles in the circuit. About 13x10⁶ PBMCs, uniformly suspended in 2 mL complete culture medium, are loaded 3D bioreactor 10 at the three-way Stopcock D at the rate of 0.2 mL/min using a syringe driven by the peristaltic pump **38.** After completing the cell loading, the pump starts to drive oscillation media flow by first pushing the medium upward in the bioreactor for 10 minutes at the rate of 0.1 mL/min, following pushing the media downward inside the bioreactor for 10 minutes at the same rate of 0.1 mL/min. The upward/downward oscillation flow cycles are repeated overnight or around 16 hours. The oscillation media flow moves the cells up and down inside the 3D bioreactor **10** to facilitate the interactions between cells and the antibody coated bioreactor surfaces to enhance T-cell activation.

After the overnight oscillation flow, the bioreactor system was switched to single-direction circulation at a relatively low flow rate of 0.04 mL/min to mimic a static condition to promote T-cell activation which results in some T-cell size expansion and clustering.

At 48-hour after T-cell activation, the G-Rex 6M is first filled with 100 mL of media and then flushed with media at a relatively high flow rate of 50 mL/min for 1 minute, disaggregating the clusters formed by activated T cells. Perfusion at a flow rate of 1 mL/min is then applied to flush and continuously remove the disassociated cells from the 3D bioreactor **10** for transfer and collection of said T cells in the G-Rex 6M. The media flowing back into the bioreactor was taken from near the top surface of the 100 mL media in G-Rex 6M in this cell free zone. The 3D bioreactor **10** was flushed again at 50 mL/min for 1 minute to dissociate any residual cells on Day 2, around 54-hours after T-cell activation. On Day 3, around 65 hours after T-cell activation, the 3D bioreactor **10** was flushed one more time. Then the 3D bioreactor was disconnected. The disconnected bioreactor and tubing were flushed by pumping through 130 mL of PBS at 50 mL /min. The residual cells were collected and added into the G-Rex 6M. The cells in the G-Rex continued to proliferate, achieving 5-7 population doublings.

An anti-CD3 anti-CD28 coated Dynabeads^{™} based T-cell activation and expansion experiment was performed at the same time as the control. Half of PBMCs (i.e., 7.0x10⁶) used in bioreactor study were used in Dynabeads^{™} experiment. The PBMCs were seeded in two wells of a 6-well plate, mixed with two times of Dynabeads^{™} in 6.5 mL of media in each well. At 65 hours after T-cell activation, the PBMCs were isolated from the Dynabeads^{™} and then transferred to G-Rex 6M for expansion.

It may therefore now be appreciated that the 3D bioreactor in combination with an expansion bioreactor may preferably be configured as a scalable automated system. As illustrated in **FIG. 7B****,** with the use of software control, such as LabVIEW^{™} software, one may program and automate the indicated steps of priming, loading PBMCs, binding of T-cells to the 3D bioreactor, activating and transduction to form CAR T-cells, dissociating the CAR T-cells and transfer of the dissociated cells to the expansion bioreactor (G-Rex Expansion) and proliferation of the CAR T-cells in the expansion bioreactor followed by CAR T-cell harvesting.

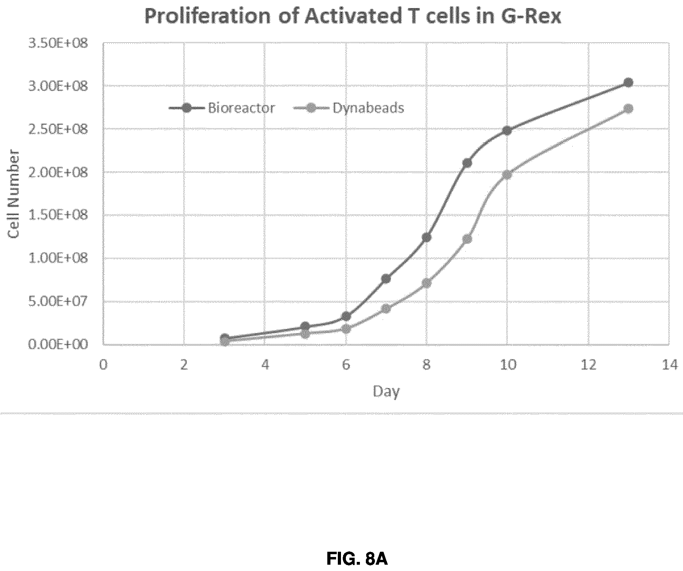

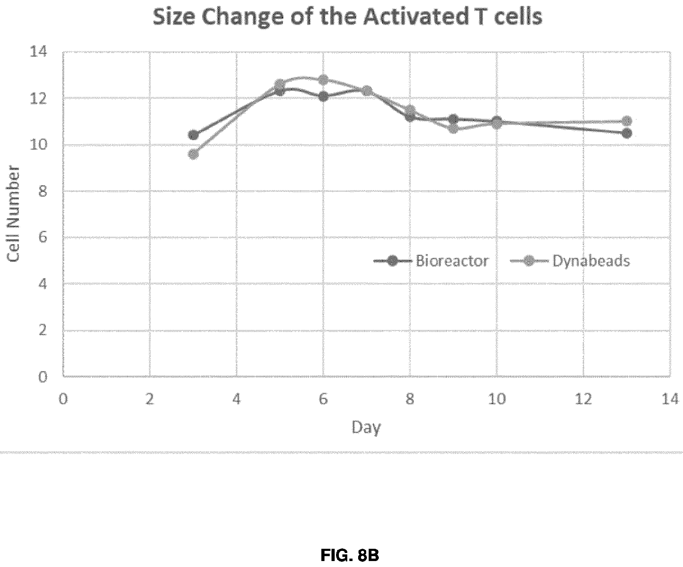

**Table 2** below shows the cell population profile as a function of time during the 3D bioreactor and Dynabeads^{™} experiments noted above. **FIG. 8A** illustrates the change in cell numbers for the 3D bioreactor versus the use of the Dynabeads^{™} over the indicated time period. **FIG. 8B** illustrates the average cell size over the indicated period. The data shows the proliferation of the activated cells in both the 3D bioreactor and Dynabeads^{™} experiments.

**Table 2**

| **Measurement of Cells in G-Rex After Activation Comparing 3D Bioreactor To Dynabeads^{™}** | | | | | | |
|---|---|---|---|---|---|---|
| | **3D Bioreactor Activation/Expansion** | | | **Dynabeads^{™} Activation/Expansion** | | |
| **Culture Time** | **Cell Number** | **Cell Size (µm)** | **Viability** | **Cell Number** | **Cell Size (µm)** | **Viability** |
| **Day 0:** PBMCs load into bioreactor Or dishes with Dynabeads^{™} | 12.6x10⁶ | 9.2 | 93.2% | 6.3x10⁶ | 9.2 | 93.2% |
| **Day 1:** | Oscillation flow | | | Mix with beads in 1:1 ratio | | |
| **Day 2:** - cells in G-Rex after second bioreactor flushing | | 10.5 | 96.7% | | | |
| **Day 3:** - cells transfer from bioreactor or dishes to G-Rex at 65 hours after activation | Residual cells in bioreactor that added into G-Rex | | | Cells isolated from beads and transferred to G-Rex | | |
| | 1.6x10⁶ | 10.2 | 91.6% | 3.6x10⁶ | 9.6 | 98.2% |
| **Day 3:** - cells in G-Rex | 7.2x10⁶ | 10.4 | 98.2% | 3.6x10⁶ | 9.6 | 98.2% |
| **Day 5:** - cells in G-Rex | 20.7x10⁶ | 12.3 | 99.1% | 12.7x10⁶ | 12.6 | 98.6% |
| **Day 6:** - cells in G-Rex | 32.9x10⁶ | 12.1 | 98.2% | 18.4x10⁶ | 12.8 | 98.2% |
| **Day 7:** - cells in G-Rex | 76.2x10⁶ | 12.3 | 99.5% | 41.3x10⁶ | 12.3 | 99.8% |
| **Day 8:** - cells in G-Rex | 125.0x10⁶ | 11.2 | 99.9% | 71.0x10⁶ | 11.5 | 99.6% |
| **Day 9:** - cells in G-Rex | 211.0x10⁶ | 11.1 | 99.6% | 122.0x10⁶ | 10.7 | 99.5% |
| **Day 10:** - cells in G-Rex | 248.0x10⁶ | 11.0 | 99.4% | 198.0x10⁶ | 10.9 | 99.7% |
| **Day 13:** - cells in G-Rex | 304.0x10⁶ | 10.5 | 99.6% | 274.0x10⁶ | 11.0 | 99.1% |
| **Day 13:** - cells harvested from the G-Rex | 294.0x10⁶ | 9.8 | 97.0% | 260.0x10⁶ | 10.2 | 97.2% |

**Table 3** next summarizes the flow cytometry analysis of the cells harvested after 13-days of T-cell activation and expansion in the 3D bioreactor and Dynabeads^{™} experiments. The data demonstrates the expansion of CD4+ and CD8+ T cells. The PBMCs activated and expanded using Dynabeads^{™} had a relatively higher fold increase than that in the 3D bioreactor system. In both experiments, the harvested cells reach the T cell purity of greater than 92%, significantly increased from the 46.2% in the original PBMCs. The CD4+ and CD8+ ratio is better in the harvested cells from the 3D bioreactor, closer to the more desirable 1:1 ratio. Most of the monocytes were captured by the bioreactor or the 6-well plate during T cell activation. Minimal B cells (< 1%) and NK cells (< 2%) are present in the harvested cells after T-cell activation and expansion.

**Table 3: Comparison of PBMC Composition/Surface Markers**

| | **Seeding PBMCs** | | **PBMCs harvested after 13-day activation/expansion within bioreactor** | | **PBMCs harvested after 13-day activation/expansion with Dynabeads^{™}** | |
|---|---|---|---|---|---|---|
| **Total harvested PBMCs number/viability/size** | 12.6x10⁶ / 6.3x10⁶ | | 294.0x10⁶ | | 260.0x10⁶ | |
| | 93.2% viability | | 97% viability | | 97.2% viability | |
| | 9.2 um | | 9.8 um | | 10.2 um | |
| CD2+ T cells | 69.7% | | 99.2% | | 98.8% | |
| CD4+ T cells | 44.9% | 46.2% | 44.2% | 92.7% | 32.9% | 92.7% |
| CD8+ T cells | 21.3% | | 49.2% | | 60.9% | |
| CD4+CD8+ fold of expansion | | | 46.8 | | 82.8 | |
| CD4+:CD8+ ratio | 2.1:1 | | 0.9:1 | | 0.54:1 | |
| CD19+ B cells | 16.1% | | 0.1% | | 0.4% | |
| CD14+ monocytes | 0.3% | | 0.0% | | 0.1% | |

### Working Example 4 - Testing of Negative 3D Bioreactor In Combination With G-Rex Cell Expansion Device For T Cell Purification, Activation, Transduction and Expansion

An antibody functionalized 1µm silica particle coated negative 3D bioreactor as described above, having a surface area of 250 cm², was next tested for T-cell transduction in addition to T-cell activation in the same perfusion system connected to a G-Rex 6M. In this experiment, the lentiviral vectors encoded to produce green fluorescent protein (GFP-LVV), at the concentration of 13.6E+06 TU/mL, were used to transduce the T cells. At 24 hours after T-cell activation, 0.5 mL of the GFP-LVV, were first diluted to 2 mL, and then injected into the 3D bioreactor. The same oscillation flow discussed above was used to enhance the interactions between the transducing lentiviral vectors and the T-cells bound to the bioreactor surface.

At 48 hours after the T-cell activation, or 24 hours after T-cell transduction, the 3D bioreactor system cells were released from the 3D bioreactor to the G-Rex expansion reactor. The processes are the same as discussed above. The expanded T cells were harvested on Day 7 after the experiments. **Table 4** shows the viability and proliferation of the PBMCs after activation and transduction. The cell viability is similar to the T cell activation study described above. The cell proliferation (or expansion) appears slightly higher than that in the previous T-cell activation study.

**Table 4: Measurement of Cell Viability and Proliferation in G-Rex Expansion Reactor after T cell Activation and Transduction Using The Negative 3D Bioreactor**

| | **Bioreactor Activation/Transduction /Expansion** | | |
|---|---|---|---|
| **Culture Time** | **Cell Number** | **Cell Size (µm)** | **Viability** |
| **Day 0:** PBMCs loaded into bioreactor | 12.1x10⁶ | 9.2 | 93.4% |
| **Day 1:** | Oscillation flow | | |
| **Day 2:** - start transduction | Oscillation flow | | |
| **Day 3:** - cells transfer from bioreactor to G-Rex at 65 hours after activation | Residual cells in bioreactor | | |
| | 0.6x10⁶ | 10.0 | 88.4% |
| **Day 5:** - cells in G-Rex | 35.7 x10⁶ | 11.3 | 94.6% |
| **Day 7:** - cells in G-Rex | 103 x10⁶ | 11.0 | 99.5% |
| **Day 7:** - Cells harvested from G-Rex for flow cytometry | 62.3x10⁶ | 9.3 | 97.8% |

**Table 5** next illustrates the flow cytometry analysis of the cells before seeding into and after harvesting from the 3D bioreactor. The data shows over 30% of T cells were transduced on Day 7. The T-cell population was also enriched from 50.3% to 94% after activation, transduction, and expansion. Most of the B cells and the monocytes were eliminated by the bioreactor and culture process.

**Table 5: Comparison of BPMC Composition/Surface markers After 7-day Activation, Transduction, and Expansion Using the Bioreactor/G-Rex System**

| | | Seeding PBMCs | | PBMCs harvested after 7-day activation/transduction/expansion within a bioreactor/G-Rex | |
|---|---|---|---|---|---|
| Total harvested BPMCs number/viability/size | | 12.1x10⁶ | | 62.3x10⁶ | |
| | | 93.4% viability | | 97.8% viability | |
| | | 9.2 um | | 9.3 um | |
| CD2+ T cells | | 64.0% | | 98.5% | |
| CD4+ T cells | | 31.9% | 53.4% | 30.7% | 93.3% |
| CD8+ T cells | | 21.5% | | 62.6% | |
| | CD4+ GFP+ T cells | 0 | 0% | 20.4% | 30.9% |
| | CD8+ GFP+ T cells | 0 | | 10.5% | |
| CD4+CD8+ fold of expansion | | | | 5.1 | |
| CD4+ : CD8+ ratio | | 1.48:1 | | 0.49:1 | |
| CD19+ B cells | | 9.2% | | 0.1% | |
| CD14+ monocytes | | 12% | | 0.1% | |

## Claims

1. A method for separating, activating, transducing and expansion of T-cells comprising:
a. supplying a 3D bioreactor comprising:
i. a plurality of voids having a diameter **D** and a plurality of pore openings between said voids having a diameter **d,** including a void surface area for coating, wherein 90% or more of said voids have a selected void volume (V) that does not vary by more than +/- 10.0% and 90% or more of said pore openings between said voids have a value of d that does not vary by more than +/- 10.0%; or
ii. a plurality of solid geometrical structures having outer surfaces for coating, wherein 90% or more of said solid geometrical structures have a volume (V) that does not vary by more than +/- 10.0%;
b. coating said 3D bioreactor with antibodies;
c. binding T-cells to said antibodies;
d. activating said T-cells;
e. transducing the antibody bound T-cells by a transduction reagent; and
f. removing said transduced T-cells from said 3D bioreactor and transferring to a T-cell expansion bioreactor wherein said transduced T-cells undergo expansion.

2. The method of claim 1 wherein said binding of T-cells to said antibodies comprises perfusing said 3D bioreactor with peripheral blood mononuclear cells (PBMCs).

3. The method of claim 1 wherein said transduced T-cells that are removed from said 3D bioreactor are prevented from transferring back into said 3D bioreactor.

4. The method of claim 1 wherein said 3D bioreactor comprises biocompatible material, or wherein said 3D bioreactor void surface area or geometrical structure outer surfaces are initially coated with substituted or unsubstituted poly(p-xylylene), β-casein or polydopamine prior to coating with said antibodies.

5. The method of claim 1 wherein said coating of said 3D reactor with antibodies comprises coating of said 3D bioreactor with antibody labelled particles.

6. The method of claim 5 wherein said antibody labelled particles comprise particles having a particle diameter of 10 nm to 1.0 µm.

7. The method of claim 5 where said antibody labelled particles comprise silica particles, or wherein said antibody labelled particles comprise polymeric particles.

8. The method of claim 5 wherein said antibody labelled particles comprise particles coated with a biotin binding molecule wherein said biotin binding molecule is coated with biotinylated antibodies.

9. The method of claim 8 wherein said biotin binding molecule comprises a tetrameric protein, wherein said tetrameric protein preferably comprises avidin, streptavidin or deglycosylated native avidin protein.

10. The method of claim 8 wherein said biotinylated antibodies are selected from the group consisting of anti-CD3 antibody, anti-CD22 antibody, anti-CD25 antibody and anti-CD28 antibody.

11. The method of claim 1 wherein said T-cells are transduced with lentivirus vectors.

12. An apparatus for separating, activating, transducing and expansion of T-cells comprising:
a. a 3D bioreactor comprising:
i. a plurality of voids having a diameter **D** and a plurality of pore openings between said voids having a diameter **d,** including a void surface area for coating, wherein 90% or more of said voids have a selected void volume (V) that does not vary by more than +/- 10.0% and 90% or more of said pore openings between said voids have a value of d that does not vary by more than +/- 10.0%; or
ii. a plurality of solid geometrical structures having outer surfaces for coating, wherein 90% or more of said solid geometrical structures have a volume (V) that does not vary by more than +/- 10.0%; and
b. a T-cell expansion bioreactor connected to said 3D bioreactor.

13. The apparatus of claim 12 wherein T-cells in said T-cell expansion reactor are prevented from flowing into said 3D bioreactor, or wherein said 3D reactor comprises biocompatible material.

14. The apparatus of claim 12 wherein said 3D bioreactor is coated with antibodies wherein said coating with antibodies preferably comprise antibody labelled particles.

15. The apparatus of claim 12 wherein said void surface area or said geometrical structure outer surfaces are coated with substituted or unsubstituted poly(p-xylylene), β-casein or polydopamine.
